# EUROPEAN PATENT SPECIFICATION

(11) **EP 2 536 850 B1**
(45) Date of publication and mention of the grant of the patent: **28.12.2016**
(21) Application number: 11713351.2
(22) Date of filing: 15.02.2011
(51) Int. Cl.: C12Q 1/68

(54) **METHOD FOR DETECTING FUNGAL PATHOGENS**
VERFAHREN FÜR DEN NACHWEIS VON PILZERREGERN
PROCÉDÉ DE DÉTECTION DE PATHOGÈNES FONGIQUES

(30) Priority: 15.02.2010 IN DE03182010
(43) Date of publication of application: 26.12.2012
(73) Proprietor: Council of Scientific & Industrial Research, New Delhi 110 001 (IN)
(72) Inventor: THAKUR, Karnika, Palampur 176 061 Himachal Pradesh (IN); JHA, Gopaljee, Palampur 176 061 Himachal Pradesh (IN)
(74) Representative: Grünecker Patent- und Rechtsanwälte PartG mbB
(86) International application number: PCT/IB2011/000278
(87) International publication number: WO 2011/098907

(56) References cited:
- EP-A1- 2 130 928
- WO-A2-01/96612
- WO-A2-2010/039819

### SEQUENCE LISTING

<110> council of Scientific and Industrial Research
<120> METHOD FOR DETECTING FUNGAL PATHOGENS
<130> 318DEL2010
<160> 7
<170> PatentIn version 3.5
<210> 1
   <211> 21
   <212> DNA
   <213> Fungi
<400> 1
   cgatgaagaa cgcagcgaaa t 21
<210> 2
   <211> 23
   <212> DNA
   <213> Fungal rDNA Primer
<400> 2
   tatgcttaag ttcagcgggt atc 23
<210> 3
   <211> 1416
   <212> DNA
   <213> venturia inaequalis gi157325656
<400> 3
   cttccgtagg tgaacctgcg gaaggatcat taatgggtta gccttcgggc gcatccccac      60
   cctctgtaac cgcggcccgg attcggcgcc tggcgggacc accccccgtt tgcgaggggc     120
   cccgcctgcc ggaattagcc aaccctgcct ggaaaattga agtctgagaa caagttaaat     180
   aacacaaaac tttcaacaac ggatctcttg gttctggcaa cgatgaagaa cgcagcgaaa     240
   tgcgataagt aatgtgaatt gcagaattca gtgaatcatc gaatctttga acgcacattg     300
   cgccccctgg tattccgggg ggcacacctg ttcgagcgcc atttctaccc tggagccctg     360
   ctctgtgatg ggccccgtcc tcgcggacgg gcccgaaacc cgtgggcgcc gtcgtccggc     420
   cctgagcgta gcaagagaaa tccctcgctc ggagtgtctg tcggcaggcc gccccgaaac     480
   ccatatttac aaggttggcc tcggatcagg tggggatacc cgctgaactt aagcatatca     540
   ataagcggag gaaaagaaac caacagggat tgcctcagta acggcgagtg aagcggcaac     600
   agctcaaatt tgaaatctgg cgcaagcccg agttgtaatt tgcagaggat gtttcgggtg     660
   aagccacggt ccaagtccct tggaacagga cgtcatagag ggtgagaacc ccgtacctgg     720
   ccgtcggtgc cccccgctgt gaaactcctt cgacgagtcg agttgtttgg gaatgcagct     780
   ctaaattggt ggtaaattcc atctaaagct aaataccggc cagagaccga tagcgcacaa     840
   gtagagtgat cgaaagatga aaagcacttt gaaaagagag ttaaaaagta cgtgaaattg     900
   ttgaaaggga agcgcttgcg gccagacttg cccgcggttg ctcagcccgc cttttggcgg     960
   gtgcactctt ccgcggtcag gccagcatcg gtttgggcgg tcggataaag gcgtcgggaa    1020
   cgtggcctcc cctcggggag gtgttatagc ccggcgcgca atgcggccag cccggaccga    1080
   ggttcgcgct ttgctaggat gctggcgtaa tggccgtaag cggcccgtct tgaaacacgg    1140
   accaaggagt ctaacatcta tgcgagtgtt tgggtgtcaa acccgtgcgc gtaatgaaag    1200
   tgaacggagg tgggaaccgc aaggtgcacc atcgaccgat cctgatgtct tcggaaggat    1260
   ttgagtaaga gcatagctgt tgggacccga aagatggtga actatgcgtg aatagggtga    1320
   agccagagga aactctggtg gaggctcgca gcggttctga cgtgcaaatc gatcgtcaaa    1380
   tttgcgtata ggggcgaaag actaatcgaa ccattt                               1416
<210> 4
   <211> 536
   <212> DNA
   <213> Alternaria alternata GU325663.1
<400> 4
   caatatgagg cgggctggat ctctcggggt tacagccttg ctgaattatt cacccttgtc    60
   ttttgcgtac ttcttgtttc cttggtgggt tcgcccacca ctaggacaaa cataaacctt   120
   ttgtaattgc aatcagcgtc agtaacaaat taataattac aactttcaac aacggatctc   180
   ttggttctgg catcgatgaa gaacgcagcg aaatgcgata agtagtgtga attgcagaat   240
   tcagtgaatc atcgaatctt tgaacgcaca ttgcgccctt tggtattcca aagggcatgc   300
   ctgttcgagc gtcatttgta ccctcaagct ttgcttggtg ttgggcgtct tgtctctagc   360
   tttgctggag actcgcctta aagtaattgg cagccggcct actggtttcg gagcgcagca   420
   caagtcgcac tctctatcag caaaggtcta gcatccatta agcctttttt caacttttga   480
   cctcggatca ggtagggata cccgctgaac ttaagcatat caataagccg gaggaa       536
<210> 5
   <211> 557
   <212> DNA
   <213> Glomerella cingulata 77378053
<400> 5
   tctccgttgg tgaaccagcg gagggatcat tactgagttt acgctctaca accctttgtg    60
   aacataccta taactgttgc ttcggcgggt agggtctccg cgaccctccc ggcctcccgc   120
   ctccgggcgg gtcggcgccc gccggaggac aaccaaactc tgatttaacg acgtttcttc   180
   tgagtggtac aagcaaataa tcaaaacttt taacaacgga tctcttggtt ctggcatcga   240
   tgaagaacgc agcgaaatgc gataagtaat gtgaattgca gaattcagtg aatcatcgaa   300
   tctttgaacg cacattgcgc ccgccagcat tctggcgggc atgcctgttc gagcgtcatt   360
   tcaaccctca agctctgctt ggtgttgggg ccctacacct gatgtaggcc ctcaaaggta   420
   gtggcggacc ctctcggagc ctcctttgcg tagtaacttt tacgtctcgc actgggatcc   480
   ggagagactc ttgccgtaaa acccccaatt ttccaaaggt tgacctcgga tcaggtagga   540
   atacccgctg aacttaa                                                  557
<210> 6
   <211> 1481
   <212> DNA
   <213> Colletotrichum acutatum 17426655
<400> 6
   ccttagatgt tctgggccgc acgcgcgtta cactgacgga gccagcgagt tctcccttgg    60
   ccggaaggcc cgggtaatct tgttaaactc cgtcgtgctg gggatagagc attgcaatta   120
   ttgctcttca acgaggaatc cctagtaagc gcaagtcatc agcttgcgtt gattacgtcc   180
   ctgccctttg tacacaccgc ccgtcgctac taccgattga atggctcagt gaggctttcg   240
   gactggccca gagaggcggg caaccgccac tcagggccgg aaagttatcc aaactcggtc   300
   atttagagga agtaaaagtc gtaacaaggt ctccgttggt gaaccagcgg agggatcatt   360
   actgagttac cgctctataa ccctttgtga acatacctaa ccgttgcttc ggcgggcagg   420
   ggaagcctct cgcgggcctc ccctcccggc gccggccccc accacgggga cggggcgccc 480
   gccggaggaa accaaactct atttacacga cgtctcttct gagtggcaca agcaaataat 540
   taaaactttt aacaacggat ctcttggttc tggcatcgat gaagaacgca gcgaaatgcg 600
   ataagtaatg tgaattgcag aattcagtga atcatcgaat ctttgaacgc acattgcgct 660
   cgccagcatt ctggcgagca tgcctgttcg agcgtcattt caaccctcaa gcaccgcttg 720
   gttttggggc cccacggcac acgtgggccc ttaaaggtag tggcggaccc tcccggagcc 780
   tcctttgcgt agtaactaac gtctcgcact gggattcgga gggactcttg ccgtaaaacc 840
   cccaaatttt ttacaggttg acctcggatc aggtaggaat acccgctgaa cttaagcata 900
   tcaataagcg gaggaaaaga aaccaacagg gattgcccca gtaacggcga gtgaagcggc 960
   aacagctcaa atttgaaatc tggccccagg cccgagttgt aatttgcaga ggatgctttt 1020
   ggcgcggtgc cttccgagtt ccctggaacg ggacgccata gagggtgaga gccccgtacg 1080
   gttggacacc aagcctttgt aaagctcctt cgacgagtcg agtagtttgg gaatgctgct 1140
   ctaaatggga ggtacatttc ttctaaagct aaatattggc cagagaccga tagcgcacaa 1200
   gtagagtgat cgaaagatga aaagcacttt gaaaagaggg ttaaacagca cgtgaaattg 1260
   ttaaaaggga agcgcttgtg accagacttg cgtccggtga atcacccagc tctcgcggct 1320
   ggggcacttc gccggcacag gccagcatca gcttgccgtc ggggacaaaa gcttcaggaa 1380
   cgtggctcct ctcggggagt gttatagcct gttgcataat acccttcggc gggctgaggt 1440
   acgcgctccg caaggatgct ggcgtaatgg tcatcagcga c 1481
<210> 7
   <211> 539
   <212> DNA
   <213> Botrytis cinerea/Botryotinia fuckeliana 269856246
<400> 7
   tccgtaggtg aacctgcgga aggatcatta cagagttcat gcccgaaagg gtagacctcc 60
   cacccttgtg tattattact ttgttgcttt ggcgagctgc cttcgggcct tgtatgctcg 120
   ccagagaata ccaaaactct ttttattaat gtcgtctgag tactatataa tagttaaaac 180
   tttcaacaac ggatctcttg gttctggcat cgatgaagaa cgcagcgaaa tgcgataagt 240
   aatgtgaatt gcagaattca gtgaatcatc gaatctttga acgcacattg cgccccttgg 300
   tattccgggg ggcatgcctg ttcgagcgtc atttcaaccc tcaagcttag cttggtattg 360
   agtctatgtc agtaatggca ggctctaaaa tcagtggcgg cgccgctggg tcctgaacgt 420
   agtaatatct ctcgttacag gttctcggtg tgcttctgcc aaaacccaaa tttttctatg 480
   gttgacctcg gatcaggtag ggatacccgc tgaacttaag catatcaata agcggagga 539

## Description

The following specification particularly describes the invention and the manner in which it is to be performed:

### Field of the invention:

The present invention relates to a set of universal primers useful for detection and identification of fungal pathogens.

The pathogen detection system developed in this study will be useful in distinguishing fungal pathogens, detecting fungal pathogens present in orchards even during asymptotic stage, specifying the need base spray of fungicides and also predicting the estimates of crop productions as severe yield losses are caused by various fungal diseases Furthermore, it will be useful for quarantine departments for routine monitoring of samples being imported and exported.

### Background and Prior art of the invention:

There are several methodologies available to detect fungal pathogens of plants and animals. Most of these methodologies had explored conservation and variability of fungal rDNA sequences for detecting the pathogens. The rDNA is comprised of coding 18S, 5.8S and 28S RNAs; interspersed with two non-coding ITS sequences namely ITS1 and ITS2 (White et al. 1990; PCR Protocols: A Guide to Methods and Applications. Academic Press, Inc., San Diego Pages 315-322). Specific primer pairs targeting ITS regions had been used to detect a particular pathogen and such primers in combination with group specific universal primers had been used to distinguish a limited group of pathogens. **Reference may be made to** the US patent 6080543 which has exploited this variability in the ITS region to distinguish *Eutypella vitis, Eutypa lata, Phomopsis viticola* and *Diplodia gossypina* fungal pathogens of grape plant. The specific primers had been used to distinguish presence of *Pyrenophora tritici-repentis* and *Pyrenophora teres* (US Patent 6358680) and *Monilinia laxa* and *Monilinia fructicola* (US Patent 6221595). US Patent application 20030099975 describes a PCR assay for detection of *Alternaria* spp., *Cladosporium carpophilum* and *Colletotrichum acutatum* wherein specific primers are used to distinguish these pathogens. The detection of a pathogen by use of specific primer had also been explored to detect contamination in food products. For example, WIPO Patent application Wo/2000/046397 describes a technique to specifically detect Alternaria in food products. The US Patent 6858387 describes use of combination of species specific primers and universal primers to detect several fungal species. In US patent application 20080227108, the inventors distinguish different species of *Aspergillus* present in the sample by calculating the sequence variability in the PCR amplified region of the ITS region. **The drawbacks** of the above mentioned techniques are that they are dependent on pathogen specific primers for identification of pathogens. Several studies had focused on designing pathogen specific probes and explored them in hybridization and microarray based assays to detect and distinguish various fungal pathogens present in the sample. **Reference may be made to** the following patents US Patent 6372430, EP0422872A2, EP0422873B1, WO/1998/055649A1, WO/1996/021741A1, US Patent 5519127, US Patent 5707802, US Patent 5426027 etc. in this regard. Sholberg et al. 2005 (Plant Dis. 89:1143-1150) have developed a DNA macroarray wherein oligonucleotides corresponding to the spacer regions of ITS sequences of the fungal pathogens *Penicillium expansum, Botrytis cinerea, Podosphaera leucotricha, Venturia inaequalis* and *Erwinia amylovora* (a bacterial pathogen) of apple were fabricated on a nylon membrane. The authors have successfully detected these pathogens and differentiated them from closely related species. Although the hybridization and microarray based techniques allow user to detect several pathogens at a time, however **the major drawbacks are** that these techniques are technically sensitive, time consuming, costly and dependent on the availability of specific probe for each of the pathogen to be detected.

Beside these methodologies, the robustness of PCR-RFLP had also been explored in various studies to distinguish fungal pathogens. ITS specific PCR followed by RFLP analysis had been shown to accurately distinguish the *Colletotrichem gloeosporioides* from *C*. *capsici* (Tapia-Tussell et al. 2008; Mol Biotechnol. 40:293-8.) and *Venturia nashicola* from *V. pirina* (Le Cam et al. 2001; Phytopathology 91:900-904). RFLP analysis of PCR products obtained using combination of ITS specific universal primer and basiodiomycetes specific primer could differentiate basiodiomycetes causing spruce wood white rot and brown rot diseases (Jasalavich et al. 2000; Applied and environmental microbiology. 4725-4734). PCR-RFLP analysis could distinguish *Eutypa lata* (vascular cancer pathogen) from the grapevine wood trunk pathogens (*Phomopsis viticola, Botryodiplodia* sp., *Phaeoacremonium aleophilum* and *Phaeomoniella chlamydospora)* and several other *Eutypa sp* (Rolshausen et al. 2004; Plant Dis. 88:925-929). The technique had also been used to distinguish Botrytis species causing neck rot disease of onion (Nielsen et al. 2002; Plant Dis. 86:682-686), differentiate some *Eucalyptus* foliar diseases causing *Mycoshaerella* species (Kularatne et al. 2004; Mycol. Res. 108:1476-1493) identify variations (both inter and intra species) in *Eucalyptus* associated ectomycorrhizal fungi (Glen et al. 2001; Mycological research.105:843-858) and also detect six medically important Candida species (Mirhendi et al. 2006;. J. Med. Mycol. 47:225-229). **Reference may be made to** US patent 5780271 which describe use of PCR-RFLP to distinguish phytophthora species. The 600 bp PCR amplicon indicates the presence of phytophthora species in the sample which upon digestion with *Hae*III restriction enzyme generates polymorphism to distinguish the *P. cactorum* from *P. infestans.* Using the fungal ITS specific universal primer along with the SBFS (Apple sooty blotch and Flyspeck complex) primers, Duttweiler et al. 2008 (Plant Dis. 92: 794-799) could obtain PCR amplicons which upon digestion with *Hae*III restriction enzyme could differentiate the SBSF associated pathogenic fungi upto genus level. Similarly, PCR amplification of ITS region followed by RFLP analysis could distinguish *Penicillium* species which causes blue mold disease of apple (Pianzzola et al. 2004; Plant Dis. 88:23-28). The major existing **drawback of** the available PCR-RFLP based techniques is that they are mostly detecting limited number/groups of pathogens.

### Patents References:

| | | | |
|---|---|---|---|
| EP0422872A2 | Nucleic acid probes and methods for detecting fungi | Apr,2006 | Weisburg *et al.* |
| EP0422873B1 | Nucleic acid probes and methods for detecting *Cryptococcus neoformans* | May,2000 | Weisburg *et al.* |
| US 6858387 | Nucleic acid probes and methods for detecting clinically important fungal pathogens | Feb,2005 | Smith *et al.* |
| US 5426027 | Nucleic acid probes and methods for detecting *Candida* DNA cells in blood | June, 1995 | Lott *et al.* |
| US 5519127 | Nucleic acid probes for the detection of *Pneumocystis Carnii* | May,1996 | Shah *et al.* |
| US 5707802 | Nucleic acid probes for the detection and identification of fungi | Jan, 1998 | Sandhu *et al.* |
| US 5780271 | PCR assays for *Phytophthora* species | Jul,1998 | Ristaino |
| US 6080543 | Detection of fungal pathogens | Jun,2000 | Engel *et al.* |
| US 6221595 | Detection of *Monilinia* spp. Using polymerase chain reaction | Apr,2001 | Beck *et al.* |
| US 6358680 | Detection of wheat and barley fungal pathogens using the PCR | Mar,2002 | Beck *et al.* |
| US 6372430 | Nucleic acids for detecting *Aspergillus* species and other filamentous fungi | Apr,2002 | Morrison *et al.* |
| US application 20030099975 | Detection of fungal pathogens using the polymerase chain reaction | May, 2003 | Barnett *et al.* |
| US application 20080227108 | Molecular identification of *Aspergillus* species | | |
| WO/11996/021741A1 | Nucleic acid probes for the detection and identification of fungi | Jul, 1996 | Sandhu *et al.* |
| WO/1998/055649A1 | Nucleic acid probes for the detection and identification of fungi | Dec,1998 | Sandhu *et al.* |
| WO/2000/046397 | Nucleic acid based assay and | Aug,2000 | Kashi *et al.* |
| | kit for the detection of alternaria contamination in food products | | |

### Objects of the invention:

**The main object of the patent invention is to design and provide** a set of universal primers which amplifies ITS region of fungal rDNA sequences useful for detecting fungal pathogens.

**Another object of the present invention is** to provide a method for detection and identification of fungal pathogen comprising isolation of DNA from the fungal mycelium(s) and/or infected tissue(s), PCR amplification of their ITS2 regions using designed universal primer pairs, suitable restriction digestion enzyme, resolution of polymorphic bands by gel electrophoresis and analysis of obtained RFLP bands by PathDec Tool which compares the obtained RFLP banding pattern with the prestored pathogen specific banding patterns obtained through *insilico* analysis to detect and distinguish various fungal pathogens.

### Novelty and inventive step(s) of the present invention with respect to the prior art:

Various PCR based methodologies had been devised to detect various fungal pathogens of the plant and the animals. Most of these techniques have been developed to detect a particular pathogen or a limited number of pathogens at a time. Specific primer pairs targeting the ITS regions of pathogens had been used to detect a particular pathogen and such primers in combination with group specific universal primers had been used to distinguish a small number of pathogens. Our innovation of designing ITRRF1 and ITRRR1 universal primers, PCR amplification using these primers, restriction digestion of the PCR products with *HinF*I enzyme, visualization of gel, and analysis of the restriction fragments through PathDec, PERL based Tool leads to identification and detection of most of fungal pathogens, even before visual appearance of disease symptoms in less than 6 hours time. The PathDec assists users to analyze complex RFLP banding pattern even without use of specific image processing Tool/instrument to identify and differentiate fungal pathogens present in the sample.

### Brief description of figures:-

**Figure 1** **represents** PCR amplification using ITRRF1 and ITRRR1 universal fungal primers. Lane 1: *Alternaria alternata*; Lane 2: *Venturia inaequalis*; Lane 3: *Glomerrella cingulata*; Lane 4 : *Colletotrichum acutatum*; Lane 5: *Botrytis cinerea.* Lane 6: Endophytic fungus; Lane 7: 100bp ladder.
**Figure 2** **represents** PCR-RFLP analysis with *HinF*I restriction enzyme. Lane 1:20bp ladder; Lane 2: *Alternaria alternata*; Lane 3: *Venturia inaequalis*; Lane 4: *Glomerella cingulata;* Lane 5: *Colletotrium acutatum* Lane 6: *Botrytis cinerea.*
**Figure 3** **represents** PCR-RFLP analysis to distinguish mixture of three different fungal pathogens of apple. Lane 1: 20bp ladder; Lane 2: Mixture of *Alternaria alternata, Venturia inaequalis, Glomerella* leaf spot pathogen.
**Figure 4** **represents** PCR-RFLP analysis to distinguish mixture of four different fungal pathogens of apple. Lane 1: 20bp ladder; Lane 2: Mixture of *Alternaria alternata*, *Venturia inaequalis,* Glomerella leaf spot pathogen *and Botrytis cinerea.*
**Figure 5** **represents** PCR-RFLP analysis of *Alternaria alternata* infected leaf during asymptotic stage. Lane 1: 100bp ladder; Lane 2: Mock infected apple leaf; Lane 3: *Alternaria alternata* infected leaf.

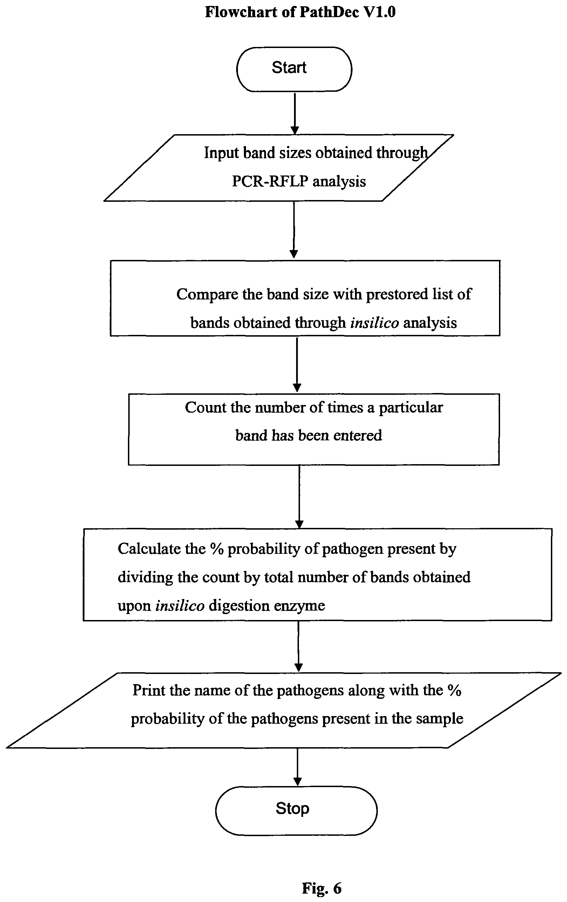

**Figure 6** Flowchart of PathDec VI.0
**Table 1 represents** *Insilico* restriction analysis of ITRRF1 & ITRRR1 primer amplifiable rDNA sequences of apple fungal pathogens with *HinF*I enzyme.
**Table 2 represents** Fungal pathogens used for validating the methodology developed in this study.

### Summary of the invention:

**Accordingly the present invention provides** a set of universal primers and a method for detection and identification of fungal pathogens. The method **comprises** DNA isolation either from fungal mycelium or symptomatic/asymptotic tissues, PCR amplification of fungal rDNA by universal fungal specific ITRRF1 and ITRRR1 primers, restriction digestion of PCR products by suitable enzyme, resolution of obtained bands by gel electrophoresis, visualization of the gel and interpretation of results by PathDec Tool but not limited to it for revealing the presence/absence of the pathogens in sample and provide their percentage probability.

**In an embodiment of the present invention** a set of universal primers having SeqID No.1 and SeqID No.2 useful for detection of fungal pathogens is disclosed.

**In another embodiment of the present invention** a set of primers having the following characteristics:
i. length of the said forward primer is 21 mer and for the reverse primer is 23 mer,
ii. G+C content is in range of 43-48%,
iii. Tm is in range of 53°C,
iv. Annealing temperature is preferably in the range of 50-60°C, optimum at 54°C is disclosed.

**In another embodiment of the present invention** a set of primers wherein their length
varies by addition or deletion of 5 nucleotides both in the forward and reverse primer in either 5' or 3' direction of the primer pair is disclosed.

**In another embodiment of the present invention** a set of primers useful for identifying unculturable fungi is disclossed.

**In yet another embodiment of the present invention** a method for identifying fungal pathogen comprising steps of:
a. designing universal primers having SeqID No. 1 and SeqID No.2
b.PCR amplification of fungal rDNA by using primers obtained in step a,
c.restriction digestion of PCR products obtained in step b using restriction enzymes selected from the group comprising of *Rsa*I, *EcoR*I, *Hind*III, *Hae*II, *Hae*III, *HinF*I, *Pho*I, *Msp*I, *Taq*I etc to generate Restriction Fragment Length Polymorphism,
d.resolution of bands obtained in step c by gel electrophoresis,
e. feeding the PCR-RFLP band information obtained in step d to PathDec tool,
f. comparing the input band information obtained in step e with the *insilico* obtained banding pattern for each of the pathogen using the algorithm wherein the database has a standard banding pattern stored for fungal pathogens
g. identifying the fungal pathogen present in the sample, is disclosed.

**In yet another embodiment of the present invention** a method for identifying fungal pathogens of apple is disclosed.

**In yet another embodiment of the present invention** a kit for detecting fungal pathogens wherein the kit comprises of:
a. Primer set having Seq. ID 1 and 2,
b. Suitable reagents for PCR,
c. Suitable restriction enzyme,
d. Instruction manual, is disclosed.

### Detailed description of the invention:

Various PCR based methodologies had been devised to detect various fungal pathogens of plants and animals. Most of these techniques have been developed to detect a particular pathogen or a limited number of pathogens. Our innovation of designing universal primers to PCR amplify the highly polymorphic region of the fungal rDNA sequences, restriction digestion of PCR products with *HinF*I enzyme and analysis of the restriction fragments through PathDec tool which compares the obtained RFLP banding pattern with the prestored pathogen specific banding patterns obtained through *insilico* analysis leads identification and detection of almost all the fungal pathogens of apple in less than 6 hours even before the visual appearance of the disease symptoms. As the primers designed in this study are universal in nature, hence it is expected that this methodology would be able to detect and distinguish fungal pathogens of other plant/animal species. However, the suitable restriction enzyme has to be identified which could generate polymorphisms and the PathDec database need to be updated for the pathogens of other plant/animals.

The various process steps involved in the present innovation are as follows: **DNA isolation:** The DNA from Apple tissues and fungal mycelium were isolated by following commonly used CTAB method (Doyle JJ, Doyle JL (1987) A rapid DNA isolation procedure for small quantities of fresh leaf tissue. Phytochem Bull 19:11-15) or using any commercial DNA isolation kit. **Designing of Universal PCR primers:** All available rDNA sequences of fungal pathogens of apple were downloaded from NCBI (http://www.ncbi.nlm.nih.gov). Multiple sequence alignment was performed using CLUSTALW2 (http://www.ebi.ac.uk/Tools/clustalw2/index.html) to find conserved regions in these sequences. One conserved stretch in 5.8S rDNA region was used for designing forward primer (ITRRF1; 5' CGATGAAGAACGCAGCGAAAT) while the reverse primer (ITRRR1: TATGCTTAAGTTCAGCGGGTATC) was designed from the second conserved stretch in such a way that the primer binding sites flank ITS2 region of the fungal rDNA sequences. ***Insilico* restriction digestion analysis to identify polymorphism amongst apple fungal pathogens:** The PCR amplifiable regions of rDNA sequences were demarcated in the downloaded rDNA sequences of apple fungal pathogens. The sequences of these regions were subjected to *insilico* restriction digestion analysis using online tool; NEB cutter V2.0 (http://tools.neb.com/NEBcutter2). Custom digestion was performed on each of these sequences using several common restriction enzymes (*Rsa*I, *EcoR*I, *Hind*III, *Hae*II, *HinF*I, *Pho*I, *Msp*I, *Taq*I) **(Table 1).** Out of these, *HinF*I digestion could reveal polymorphism in the banding pattern for 13 out of 19 apple fungal pathogens whose sequences were available in NCBI **(Table 1).** However, due to highly conserved nature of the rDNA sequences, the *Alternaria alternata, Botryosphaeria berengeriana* and *Marssonina mali* (having similar RFLP pattern: 150, 116, 53, 8) and also *Botryotinia fuckeliana, Monilinia fructicola* and *Sclerotinia sclerotiorum* (having similar RFLP pattern: 165, 89, 53, 8) could not be distinguished by the methodology developed in this innovation.

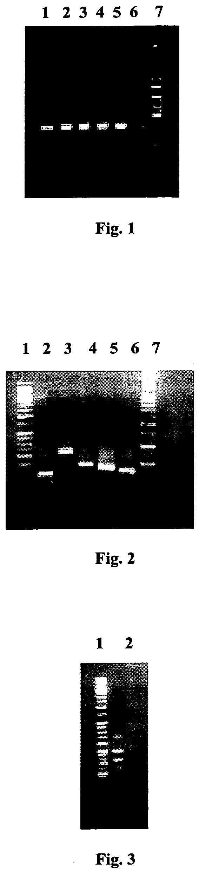

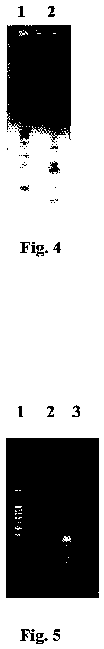

In order to verify that strain specific variations does not alter RFLP pattern of a particular pathogen, at least 4-5 rDNA sequences corresponding to different strains of each of these pathogens were downloaded from NCBI, PCR amplifiable regions were demarcated and were subjected to *insilico* restriction digestion analysis. The analysis revealed that the banding patterns are same in all the strains of particular pathogens and no strain specific variations was detected. **PCR amplification by designed universal primers:** The DNA from cultures of four different fungal pathogens of apple **(Table 2)** obtained from MTCC, Chandigarh (India) isolated following methodology described above was subjected to PCR amplifications using the ITRRF1 and ITRRR1 primers. 50 µl of PCR reaction was set by mixing 5 µl of 10X PCR buffer, 5 µl of 10X MgCl₂, 4 µl of dNTP mix, 0.4 µl of Taq (5U/µl) and 1 µl (3pm) each of ITRRF1 and ITRRR1 primers with final volume adjusted by addition of autoclaved deionized water. After initial denaturation at 95°C for 5mins, the sample was subjected to 35 thermal cycles of 94°C for 1 min, 54°C for 30 sec and 72°C for 30 sec. The final extension was performed at 72°C for 10 min. Figure 1, depicts that approximately 320bp of PCR amplicons are produced in all these cases. **RFLP analysis of the PCR products:** 1 µg of the PCR products obtained from different apple fungal pathogens as described above were subjected to RFLP analysis by digestion with 1 µl of fast digest *HinF*I (Fermentas International Inc, Ontario, Canada) at 37°C for 15 min as per the manufacturer's instructions. The digested bands were resolved by gel electrophoresis on 3% agarose gel by running at 60V for 90 mins and imaged by Gel Documentation system (Figure 2). The banding pattern was found to be similar to that obtained through *insilico* restriction analysis. Furthermore, we accessed whether the present innovation could distinguish various pathogens if present together as a mixture. In order to analyze this, mixture of genomic DNA of three and four different pathogens in equal proportions was made and subjected to PCR-RFLP analysis as described above. As shown in Figure 3 and Figure 4, PCR-RFLP could distinguish all these pathogens. Furthermore to check the efficiency of this innovation in detecting pathogens even before appearance of visual symptoms, apple leaves infected with 10 µl of *Alternaria alternata* conidia were monitored following the methodology presented in this innovation. As shown in Figure 5, the methodology could detect presence of pathogen in the sample having no visual symptom. **Interpretation of the RFLP banding pattern by PathDec Tool:** The RFLP patterns can be interpreted by using PERL based Tool, PathDec to detect and distinguish various pathogens present in the sample. The PathDec Tool could successfully distinguish the mixture of apple fungal pathogens once the obtained PCR-RFLP band sizes (as shown in Figure 4) were provided as input to the Tool.

Analysis of RFLP bands to distinguish the pathogens is tedious especially when a number of pathogens are present in the sample. A PERL based software PathDec had been developed in this innovation to interpret the PCR-RFLP banding patterns and to assist users in identifying the fungal pathogens present in the sample. Overall the detection system developed in this study had been successfully validated to distinguish several apple pathogens individually and in mixtures.

**The following examples are given by way of illustration and therefore should not be construed to limit the scope of present invention**

### EXAMPLE-1

Culturing of fungal pathogens used in this study: The following are the growth condition used for culturing fungal pathogens of apple.

### Alternaria alternata

Growth medium -PDA (Potato Dextrose Agar; Himedia); Growth condition- Aerobic Temperature: 28°C; Incubation time : 5 days

### Glomerella cingulata

Growth medium -PDA (Potato Dextrose Agar; Himedia); Growth condition- Aerobic Temperature: 25°C; Incubation time : 5 days

### Colletotrichum acutatum

Growth medium -PDA (Potato Dextrose Agar; Himedia); Growth condition- Aerobic Temperature: 25°C; Incubation time : 5 days

### Botrytis cinerea

Growth medium -PDA (Potato Dextrose Agar; Himedia); Growth condition- Aerobic Temperature: 25°C; Incubation time : 5 days

### Venturia inaequalis

Growth medium -M-98 (malt extract 10gm/L; peptone 3gm/L and agar 15gm/L; Himedia); Growth condition- Aerobic; Temperature: 20°C; Incubation time : 14 days

### EXAMPLE-2

Designing of universal fungal specific primers: The rDNA sequences of various apple fungal pathogens were downloaded from NCBI (http://www.ncbi.nlm.nih.gov). In order to fetch a conserved region in these sequences, a multiple sequence alignment using CLUSTALW2 (http://www.ebi.ac.uk/Tools/clustalw2/index.html) was performed. Both forward (ITRRF1: 5' CGATGAAGAACGCAGCGAAAT) and reverse primers (ITRRR1: TATGCTTAAGTTCAGCGGGTATC) were designed from the conserved regions which flanks the ITS2 region of the fungal rDNA sequences.

### EXAMPLE-3

Analyzing the universality of the designed primers: In order to find whether the primers designed in this study could amplify the rDNA sequences of other fungi, we downloaded the rDNA sequences of various randomly selected fungi [*Debaryomyces hansenii* strain W4682 (GQ913348); Fungal endophyte isolate 5724 (DQ979775); *Cladosporium cladosporioides* strain STE-U (AY251074); *Cladosporium cladosporioides* isolate GG6(EF104249); *Athelia bombacina* isolate AFTOL-ID 347(DQ449026); *Chaetomium globosum* isolate B221(GQ365152); Microsphaeropsis sp. HLS303(FJ770074*); Microsphaeropsis arundinis* strain PMBMDF049(FJ798603); *Dothideomycetes* sp. 11313(GQ153229); *Aspergillus flavus* strain ATCC 11497(GU256760); *Aspergillus terreus* strain ATCC 20542(GU256759); *Aspergillus niger* strain ATCC 64973(GU256750); *Aspergillus versicolor* strain ATCC 26644(GU256744); *Debaryomyces hansenii* strain W4682(GQ913348) ; *Neurospora crassa* strain ATCC MYA-4619(GU327635); *Neurospora discreta* strain ATCC MYA-4616(GU327632); *Neurospora tetrasperma* strain ATCC MYA-4615(GU327631); Neurospora sitophila(GU192459); *Neurospora crassa* strain ATCC MYA-4614(GU327630); *Penicillium sp.* GG-2009a (GQ418173); *Magnaporthe grisea* strain ATCC MYA-4617 (GU327633); *Botryosphaeria obtusa* strain SDAU07159 (FJ171717); *Penicillium expansum* strain NRRL 6069 (DQ339562); *Cladosporium herbarum* isolate wb317 (AF455479); *Schizothyrium pomi* (FJ425206); *Mycosphaerella pyri* strain CBS 100.86(EU167606); *Venturia pirina* strain ICMP (AF333439); *Podosphaera leucotricha* voucher BPI 878262 (EU148597)] and look for the presence of the both forward and reverse primer binding site. The primer binding sites were present in all these sequences suggesting the fungal specific universal nature of these primers.

### EXAMPLE-4

*Insilico* approach to distinguish various fungal pathogens: All available rDNA sequences of fungal pathogens of apple were downloaded from NCBI (http://www.ncbi.nlm.nih.gov). *Insilico* restriction digestion analysis using online tool; NEB cutter V2.0 (http://tools.neb.com/NEBcutter2) was explored to study polymorphism amongst apple fungal pathogens. The PCR amplifiable regions of rDNA sequences were demarcated in the downloaded rDNA sequences of apple fungal pathogens and were subjected to custom digestion analysis using several common restriction enzymes (*Rsa*I, *EcoR*I, *Hind*III, *Hae*II, *HinF*I, *Pho*I, *Msp*I, *Taq*I). The analysis revealed that *HinF*1 digestion could provide length polymorphism in the PCR amplicons of different apple pathogens (Table 1).

**Table 1 represents Insilico restriction analysis of ITRRF1 & ITRRR1 primer amplifiable rDNA sequences of apple fungal pathogens with HinFI enzyme**

| **Pathogen** | **Disease caused** | **Accession ID** | **Amplicon Size** | **Fragments** |
|---|---|---|---|---|
| *Alternaria mali¹* | Alternaria blotch | 15209127 | 327bp | 150,116,53,8 |
| *Armillaria mellea* | Armillaria root rot | 37785994 | 535bp | 384,90,53,8 |
| *Armillaria tabescens* | Clitocybe root rot | 37785996 | 528bp | 125,109,92,89,53, 20,20,12,8 |
| *Botryosphaeria obtusa* | Black rot, frogeye leafspot and canker | 198448293 | 334bp | 273,53,8 |
| *Botryosphaeria berengeriana¹* | Apple ring rot and canker | 189162092 | 328bp | 151,116,53,8 |
| *Botryotinia fuckeliana¹* | Gay mold rot = dry eye rot, blossom-end rot | 240129528 | 315bp | 165,89,53,8 |
| *Butlerelfia eustacei* | Fisheye rot | 4235188 | 361bp | 171,129,53,8 |
| *Colletotrium gloeosporioides²* | Glomerella leaf spot | 154125153 | 328bp | 187,80,53,8 |
| *Glomerella cingulata²* | Glomerella leaf spot | 217314830 | 328bp | 187,80,53,8 |
| *Grovesinia pyramidalis* | Zonate leaf spot | 2809016 | 282bp* | 132,89,53,8 |
| *Marssonina coronariae* | Marssonina blotch | 169672298 | 288bp* | 227,53,8 |
| *Marssonina mali¹* | Marssonina blotch | 189162088 | 327bp | 150,116,53,8 |
| *Monilinia fructicola¹* | American brown rot | 212675236 | 313bp | 163,89,53,8 |
| *Monilinia mali* | Monilia leaf blight | 48927615 | 266bp* | 205,53,8 |
| *Peltaster fructicola* | Sooty blotch complex | 218963779 | 336bp | 275,53,8 |
| *Sclerotinia sclerotiorum¹* | Calyx-end rot | 170280297 | 315bp | 165,89,53,8 |
| *Podospharea leutricha* | Powdery mildew | 26417854 | 293bp* | 189,53,43,8 |
| *Venturia inaequalis* | Apple scab | 167460230 | 320bp | 259,53,8 |
| *Xylaria mali* | Black root rot | 8809739 | 323bp | 134,128,53,8 |

| | | | | |
|---|---|---|---|---|
| *: reverse primer binding sites are not present in the available sequences at NCBI. ¹: These pathogens could not be distinguished by this method ²: Both are anamorphic. | | | | |

### EXAMPLE-5

Isolation of genomic DNA from fungal mycelium and plant tissues: 100mg of tissue (mycelium, leaves, fruits etc) were used for DNA isolation as per CTAB DNA isolation protocol (Doyle JJ, Doyle JL (1987) A rapid DNA isolation procedure for small quantities of fresh leaf tissue. Phytochem Bull 19:11-15).

### EXAMPLE-6

PCR-RFLP analysis to reveal polymorphisms amongst fungal pathogens on agarose gel: 50 µl of PCR reaction was set by mixing 5 µl of 10X PCR buffer, 5 µl of 10X MgCl₂, 4 µl of dNTP mix, 0.4µl of Taq (5U/µl) and 1µl (3pm) each of ITRRF1 and ITRRR1 primers with final volume adjusted by addition of autoclaved deionized water. After initial denaturation at 95°C for 5 mins, the sample was subjected to 35 thermal cycles of 94°C for 1 min, 54°C for 30 sec and 72°C for 30 sec. The final extension was performed at 72°C for 10 min. 1 µg of the PCR products (Figure 1) were subjected to RFLP analysis by digestion with 1 µl of fast digest *HinF*I (Fermentas International Inc, Ontario, Canada) at 37°C for 15 min as per the manufacturer's instructions. The digested bands were resolved by gel electrophoresis on 3% agarose gel by running at 60V for 90 mins and imaged by Gel Documentation system (Figure 2).

### EXAMPLE-7

Distinguishing different fungal pathogens of apple cultured on laboratory medium: The PCR was performed on the isolated DNA of different fungal pathogens (Table 2) and RFLP analysis followed by agarose gel electrophoresis as per Example 5. The analysis revealed that the methodology could distinguish all these fungal pathogens of apple (Figure 2).

**Table 2: Fungal pathogens used for validating the methodology developed in this study**

| **Fungal Pathogen** | **MTCC no.** |
|---|---|
| *Alternaria alternata* | 149 |
| *Venturia inaequalis* | 1109 |
| *Glomerella cingulata* | 2033 |
| *Colletotrichum acutatum* | 1037 |
| *Botrytis cinerea.* | 2104 |

### EXAMPLE-8

Distinguishing fungal pathogens present in a mixture: Mixture of genomic DNA of three or four different apple fungal pathogens in equal proportions was made and subjected to PCR-RFLP analysis as described in Example 5. As shown in Figure 3 and Figure 4; PCR-RFLP could distinguish all these pathogens.

### EXAMPLE-9

Use of Tool to analyze the PCR-RFLP banding patterns: analysis of PCR RFLP patterns is cumbersome specifically when number of bands has to be analyzed. PERL based Tool PathDec has been used for this purpose (Copyright protected) however the application is not limited to PathDec. The PathDec Tool could successfully distinguish the mixture of apple fungal pathogens once the obtained PCR-RFLP band sizes as described were provided as input to the Tool.

### EXAMPLE-10

Detecting fungal pathogens even before appearance of visual symptoms: Apple leaves infected with 10µl of *Alternaria alternata* conidia were monitored following the methodology as described in Example 5. The Figure 5 shows that our methodology could detect presence of pathogen in the sample having no visual symptom.

### Inference/Conclusions/Summary of observations

The present innovation describes a PCR based methodologies to detect various fungal pathogens of the plant. Our innovation of designing universal primer to PCR amplify fungal rDNA sequences, restriction digestion of PCR products with *HinF*I enzyme and analysis of obtained restriction fragments through PathDec Tool leads identification and detection of the majority of fungal pathogens of apple but not limited to apple even before visual appearance of the disease symptoms in less than 6 hours.

The main advantages of the present invention are:
1. Designing of universal primer pair to amplify fungal rDNA sequences.
2. Use of Tool to analyze and interpret PCR-RFLP data to identify and distinguish fungal pathogens.
3. Simple, robust and single protocol to detect and distinguish most of the fungal pathogens in a mixture in less than 6hr time.
4.Simple, robust and single protocol to detect and distinguish in less than 6hr time most of the fungal pathogens present in the sample even before visual appearance of disease symptoms.
5. Simple, robust and single protocol to detect and distinguish in less than 6hr time most of the fungal pathogens of apple present in the sample even before visual appearance of disease symptoms.

## Claims

1. A set of universal primers consisting of SeqID No.1 and SeqID No.2 useful for detection of fungal pathogens.

2. A set of primers as claimed in claim 1, wherein the primers have the following characteristics:
i. length of the said forward primer is 21 mer and for the reverse primer is 23 mer,
ii. G+C content is in range of 43-48%,
iii. Tm is in range of 53°C,
iv. Capable of annealing to the target nucleic acid in the preferred temperature range of 50-60°C, optimally at 54°C.

3. A set of universal primers consisting of sequences **characterised by** than their length and sequence varies from SeqID No. 1 and SeqID No. 2 by addition or deletion of 5 nucleotides both in the forward and reverse primer in either 5' or 3' direction of the primer pair, useful for detection of fungal pathogens and having characteristics ii., iii., iv. according to claim 2.

4. Use of the primers as claimed in claim 1 for identifying un-culturable fungi.

5. A method for identifying fungal pathogen comprising steps of:
a. designing universal primers having SeqID No.1 and SeqID No.2
b. PCR amplification of fungal rDNA by using primers obtained in step a,
c. restriction digestion of PCR products obtained in step b using restriction enzymes selected from the group comprising of *RsaI, EcoRI, HindIII, HaeII, HaeIII, HinFI, PhoI, MspI, TaqI* etc to generate Restriction Fragment Length Polymorphism,
d. resolution of bands obtained in step c by gel electrophoresis,
e. providing a database having a standard banding pattern stored for fungal pathogens,
f. comparing the banding pattern obtained in step d with the *in silico* obtained banding pattern obtained in step e,
g. identifying the fungal pathogen present in the sample.

6. A method for identifying fungal pathogens as claimed in claim 5, wherein the pathogens are fungal pathogens of apple.

7. A kit for detecting fungal pathogens wherein the kit comprises of:
a. Primer set consisting of SeqID No.1 and 2,
b. Suitable reagents for PCR,
c. Suitable restriction enzyme,
d. Instruction manual;

## Patentansprüche

1. Ein Satz universeller Primer bestehend aus SeqID Nr. 1 und SeqID Nr. 2, der zum Nachweis von Pilzpathogenen geeignet ist.

2. Ein Satz Primer wie in Anspruch 1 beansprucht, wobei die Primer die folgenden Merkmale haben:
i) Länge des Forward Primers ist 21 mer und des Reverse Primers ist 23mer,
ii) G+C Gehalt liegt im Bereich von 43-48%,
iii) Tm liegt im Bereich von 53 °C,
iv) Geeignet, um in dem bevorzugten Temperaturbereich von 50-60 °C an die Zielnukleinsäure anzulagern, optimal bei 54 °C.

3. Ein Satz universeller Primer bestehend aus Sequenzen, die dadurch charakterisiert sind, dass ihre Länge und Sequenz sich von SeqID Nr. 1 und SeqID Nr. 2 durch Addition oder Deletion von 5 Nukleotiden, sowohl in dem Forward als auch in dem Reverse Primer, entweder in 5' oder in 3' Richtung des Primerpaars, unterscheidet, der geeignet ist zum Nachweis von Pilzpathogenen und die Merkmalen ii), iii) und iv) gemäß Anspruch 2 hat.

4. Verwendung der Primer wie in Anspruch 1 beansprucht zur Identifizierung unkultivierbarer Pilze.

5. Ein Verfahren zur Identifizierung eines Pilzpathogens, umfassend die Schritte:
a. Entwerfen universeller Primer mit den SeqID Nr. 1 und SeqID Nr. 2
b. PCR-Amplifikation von Pilz-rDNA mittels der in Schritt a erhaltenen Primer,
c. Restriktionsverdau der PCR Produkte, die in Schritt b erhalten wurden, mittels Restriktionsenzymen ausgewählt aus der Gruppe umfassend *RsaI, EcoRI, HindIII, HaeII, HaeIII, HinFI, PhoI, MspI, TaqI* etc., um einen Restriktionsfragmentlängenpolymorphismus zu erzeugen,
d. Auftrennen der Banden, die in Schritt c erhalten wurden, mittels Gelelektrophorese,
e. Bereitstellen einer Datenbank, die ein Standardbandenmuster für Pilzpathogene gespeichert hat,
f. Vergleichen des Bandenmusters, das in Schritt d erhalten wurde, mit dem *in silico* erhaltenen Bandenmuster, das in Schritt e erhalten wurde,
g. Identifizieren des Pilzpathogens, der in der Probe vorliegt.

6. Ein Verfahren zur Identifizierung von Pilzpathogenen wie in Anspruch 5 beansprucht, wobei die Pathogene Pilzpathogene des Apfels sind.

7. Ein Kit zum Nachweis von Pilzpathogenen, wobei das Kit umfasst:
a. Einen Satz Primer bestehend aus SeqID Nr. 1 und SeqID Nr. 2,
b. Geeignete Reagenzien für eine PCR,
c. Geeignetes Restriktionsenzym,
d. Bedienungsanleitung;

## Revendications

1. Ensemble d'amorces universelles constituées de SeqID n° 1 et SeqID n° 2 utiles pour la détection de pathogènes fongiques.

2. Ensemble d'amorces selon la revendication 1, les amorces ayant les caractéristiques suivantes :
i. longueur de ladite amorce sens de 21-mer et de l'amorce anti-sens de 23-mer,
ii. teneur en G + C dans la plage de 43 à 48 %,
iii. Tm dans la plage de 53 °C,
iv. aptes à s'hybrider à l'acide nucléique cible dans la plage de température préférée de 50 à 60 °C, de manière optimale à 54 °C.

3. Ensemble d'amorces universelles constituées de séquences **caractérisées en ce que** leur longueur et séquence varient par rapport à SeqID n° 1 et SeqID n° 2 par addition ou délétion de 5 nucléotides tant dans l'amorce sens qu'anti-sens dans la direction 5' ou 3' de la paire d'amorces, utiles pour la détection de pathogènes fongiques et présentant les caractéristiques ii., iii., iv. selon la revendication 2.

4. Utilisation des amorces selon la revendication 1 pour l'identification de champignons non cultivables.

5. Procédé d'identification d'un pathogène fongique comprenant les étapes consistant à :
a. concevoir des amorces universelles ayant SeqID n° 1 et SeqID n° 2
b. amplifier par PCR un ADNr fongique en utilisant les amorces obtenues à l'étape a,
c. digérer par restriction les produits de PCR obtenus à l'étape b en utilisant des enzymes de restriction choisies dans le groupe comprenant *RsaI, EcoRI, HindIII, HaeII, HaeIII, HinFI*, *PhoI*, *MspI, TaqI,* etc. afin de générer un polymorphisme de longueur de fragment de restriction,
d. séparer les bandes obtenues à l'étape c par électrophorèse sur gel,
e. fournir une base de données dans laquelle est stocké un motif de bandes standard pour des pathogènes fongiques,
f. comparer le motif de bandes obtenu à l'étape d avec le motif de bandes obtenu *in silico* à l'étape e,
g. identifier le pathogène fongique présent dans l'échantillon.

6. Procédé d'identification de pathogènes fongiques selon la revendication 5, les pathogènes étant des pathogènes fongiques de la pomme.

7. Kit pour la détection de pathogènes fongiques, le kit étant constitué de :
a. Un ensemble d'amorces constitué des SeqID n° 1 et SeqID n° 2,
b. Réactifs appropriés pour la PCR,
c. Une enzyme de restriction appropriée,
d. Un manuel d'utilisation ;
